# EUROPEAN PATENT SPECIFICATION

(11) **EP 2 609 859 B1**
(45) Date of publication and mention of the grant of the patent: **29.04.2020**
(21) Application number: 12198962.8
(22) Date of filing: 21.12.2012
(51) Int. Cl.: A61B 6/00, A61B 6/04

(54) **Radiological apparatus**
Röntgengerät
Appareil radiologique

(30) Priority: 27.12.2011 IT CO20110077
(43) Date of publication of application: 03.07.2013
(73) Proprietor: General Medical Merate S.p.A., 24068 Seriate (IT)
(72) Inventor: Gaiani, Massimo, 20900 MONZA (IT); Guastalli, Antonio, 20847 ALBIATE (IT)
(74) Representative: Torti, Carlo Maria Emilio

(56) References cited:
- WO-A1-2006/008677
- DE-U1- 9 012 435
- US-A1- 2003 194 056
- US-A1- 2007 286 334
- US-A1- 2011 062 343

## Description

### Field of the invention

The present invention relates to a radiological apparatus.

### State of the art

In recent years. radiological apparatuses and their components have undergone significant improvements.

However, there are still many aspects which can be improved.

Among these aspects, apparatus productivity, examination execution time, time of exposure of patients and operators to X-rays, correct positioning and alignment of the X-ray emitter and X-ray detector, and flexibility of use and installation of the apparatus are worthy of mention.

When capturing a radiological image is required, it is important that the X-ray detector is aligned with the X-ray emitter.

Such an alignment is typically obtained manually, first by mechanical means, i.e. fixed and predetermined placement of both the detector and the emitter, and then by optical means, i.e. lighting of the area to be taken by the emitter.

Patent application WO 2006/008677 describes a system that guides the user in manually aligning the detector with the emitter, in particular providing him/her with information (the document does not accurately explain the nature of the information) about the position difference between the detector and the emitter; namely, the system independently detects both the position of the detector (X1, Y1, Z1, Alpha1, Beta1) and the position of the emitter (X2, Y2, Z2, Alpha2, Beta2). This solution has never been implemented on X-ray machines currently on the market, probably due to the disadvantages described below. The Applicant has analyzed this solution and believes it to be theoretically good but practically not very effective; in fact, the positioning of the detector is generally determined by six parameters: three distances and three angles; when an operator moves the detector manually it is virtually impossible that he/she independently and appropriately controls the six parameters and, therefore, despite the information received from the radiological apparatus, the alignment achievable is rather approximate, i.e. comparable with that obtainable by an experienced operator without this system. Furthermore, since capturing the radiological image takes place only once the system has deemed the alignment to be "sufficient", if the operator does not have enough experience and dexterity, no radiological image is generated, neither of good nor poor quality; in other words, this system requires considerable operator training.

### Summary of the invention

It is the general task of the present invention to improve known radiological apparatuses.

It is a first more specific purpose of the present invention to provide a radiological apparatus in which the alignment between the X-ray emitter (more precisely, the X-ray beam emitted) and the X-ray detector is facilitated.

It is a second more specific purpose of this invention to provide a radiological apparatus in which positioning of the X-ray emitter and/or X-ray detector is facilitated.

These and other purposes are achieved by the radiological apparatus having the technical features set forth in the appended claims which form an integral part of the present description.

The radiological apparatus according to the present disclosure generally comprises a patient table, an X-ray emitter mounted to an arm and adapted to be moved, an X-ray detector associated with said patient table and an electronic control unit adapted to control at least said emitter and said detector; it further comprises:
- sensors adapted to detect the actual movement of said emitter,
- at least one actuator adapted to automatically move said detector at least in a horizontal direction;
said electronic control unit is connected to said sensors and said at least one actuator, and is adapted to obtain the actual movement of said emitter from said sensors and consequently to control said at least one actuator so that, during normal use of the apparatus (i.e. when radiological images or other images are intended to be captured, for example, during a production step or an initial calibration step by an installer and/or operator), said detector is automatically aligned to said emitter before starting capturing a radiological image or a plurality of radiological images (such as in the case of a tomography).

Thanks to automatic alignment, use by the operator is highly simplified and the quality of radiological images is perfect due to precise alignment.

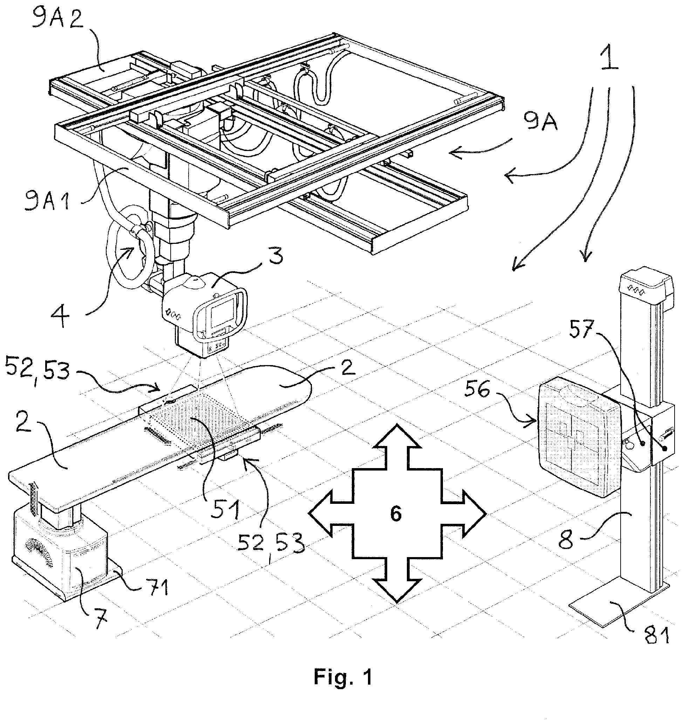

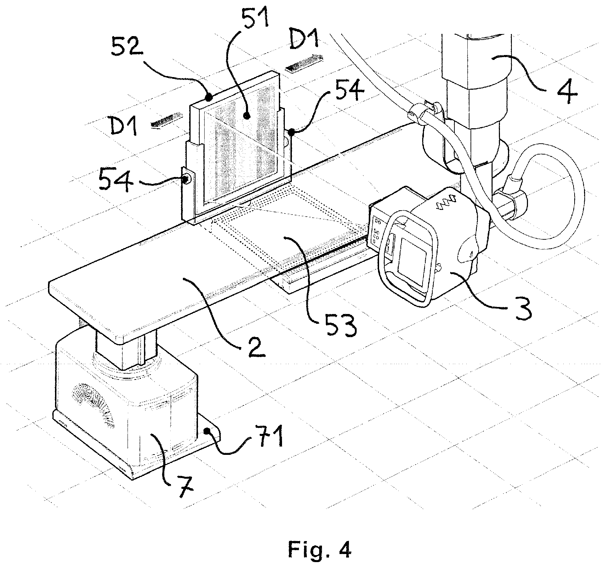

It should be noted that, according to the most advantageous embodiments of the present invention, automatic alignment is possible not only for frontal projections (see for example Fig. 1, Fig. 2, Fig. 3, Fig. 7, Fig. 10, Fig. 11, Fig. 12), often referred to as "anteroposterior", but also lateral projections (see for example Fig. 4, Fig. 5, Fig. 6); should a teleradiography system be included, automatic alignment is also advantageously applied thereto.

Once the emitter has been positioned by an operator, the apparatus is almost immediately ready to capture an image and the risk of significant patient movements is minimal.

It should be noted that the automatic movement of a detector is not a novelty per se; for example, the detector is automatically moved when the images of the "tomographies" (see for example patent application US 2011/062343 with regard to "tomosynthesis", which should not to be confused with the much more complex and accurate "computerized tomography").

The present invention has advantageous technical features; the main ones are summarized below.

Said emitter is adapted to undergo a displacement by an operator, either manually or in a motorized manner, before starting capturing a radiological image or a plurality of radiological images (such as in the case of a tomography).

Said automatic alignment typically follows said operator displacement. Possibly and advantageously, said automatic alignment immediately follows said operator displacement; i.e. the detector follows the emitter.

Said patient table may be advantageously mounted to a base, said base being adapted to move said patient table only in the vertical direction (D3) (and thus not in the horizontal direction), and therefore the position of said patient table can only be vertically adjusted.

The apparatus may advantageously comprise two actuators adapted to automatically move said detector in a first horizontal direction and in a second horizontal direction, respectively; in this case, said second direction is perpendicular to said first direction, and said electronic control unit is connected to said sensors and to said first and second actuators, and is adapted to obtain the movement of said emitter from said sensors, and consequently to control said first and second actuators such that said detector is aligned with said emitter.

Said first direction may advantageously correspond to the longitudinal direction of said patient table and said second direction may advantageously correspond to the transverse direction of said patient table.

Said patient table may be associated with a first movable support adapted to support said detector in the horizontal position; in this case, said first support comprises a sensor adapted to detect the presence of a detector supported horizontally, and to notify it to said electronic control unit to enable automatic alignment of said detector and of said emitter.

Said patient table may be associated with a first movable support adapted to support said detector in the vertical or oblique position; in this case, said first support comprises a sensor adapted to detect the presence of a detector supported vertically or obliquely, and to notify it to said electronic control unit to enable automatic alignment of said detector and of said emitter.

Said first support may have a predetermined number of stable angular positions for said detector and be adapted to notify the angular position taken by said detector to said electronic control unit to allow automatic alignment of said detector and of said emitter.

Said first support may be adapted to rotate, in particular about a vertical axis, said detector under the control of said electronic control unit to allow automatic alignment of said detector and of said emitter.

The apparatus may comprise a second movable support adapted to support said detector in the vertical or oblique position; said second support is separate and remote from said patient table; in this case, said second support comprises a sensor adapted to detect the presence of a supported detector and to notify it to said electronic control unit to enable automatic alignment of said detector and of said emitter.

Said second support may be adapted to translate, in particular in a vertical direction, said detector under the control of said electronic control unit to allow automatic alignment of said detector and of said emitter.

Said second support may be adapted to rotate, in particular about a vertical and/or horizontal axis, said detector under the control of said electronic control unit to allow automatic alignment of said detector and of said emitter.

Said second support may have a plurality of stable (vertical and/or angular) positions for said detector and be adapted to notify the position taken by said detector to said electronic control unit to allow automatic alignment of said detector and of said emitter.

Said logical control unit may be advantageously arranged such that, during normal use of the apparatus, it achieves movements of said detector such that said detector follows (possibly with a slight delay) said emitter before starting capturing a radiological image or a plurality of radiological images (such as in the case of a tomography).

Said logical control unit may be arranged to achieve the movement of said detector such that alignment between said detector and said emitter is achieved on the basis of a user command and/or when said emitter is stationary.

Said arm may be advantageously adapted to be moved in a manual and/or motorized manner.

Said arm may be adapted to be moved in an automatic manner under the control of said electronic control unit to allow automatic alignment of said detector and of said emitter. This possibility is advantageously used to achieve only a coarse alignment of said detector and of said emitter.

Said arm may comprise a first extendible arm portion, a second arm portion perpendicularly mounted to one end of said first portion with respect to said first portion; in this case, said second portion is adapted to rotate about said first portion, and said emitter is rotatably mounted to one end of said second portion. Said patient table may be mounted to a base, said base having a single foot at one end of said patient table.

Alternatively, said patient table may be mounted to a base, said base having two feet at two ends of said patient table.

The apparatus may comprise a frame adapted to support said arm at one end thereof; said frame may comprise a first frame portion adapted to be secured to a ceiling and a second frame portion mounted to said first portion in a sliding manner according to a first horizontal direction; in this case, said arm is mounted to said second frame portion in a sliding manner according to a second horizontal direction, and said second direction is perpendicular to said first direction.

The apparatus may comprise a frame adapted to support said arm at one end thereof; said frame may comprise a column, a first crosspiece and a second crosspiece; in this case, said first crosspiece is adapted to be horizontally secured to the bottom of a wall or to the floor, said second crosspiece is adapted to be horizontally secured down said wall or ceiling, said column is vertically mounted to said first crosspiece and second crosspiece in a sliding manner, and said arm is mounted to said column in a sliding manner.

### Brief description of the drawings

The technical features of the present invention as well as its advantages will become more apparent from the following description to be considered in conjunction with the accompanying drawings, in which:
Fig. 1 schematically shows a perspective view of a first embodiment of the radiological apparatus according to the present invention,
Fig. 2 schematically shows the apparatus in Fig. 1 in which the possibilities of movement of the table and of the detector associated with the table in a first operating condition are highlighted,
Fig. 3 schematically shows the apparatus in Fig. 1 in which the possibilities of movement of the table and of the detector associated with the table in a second operating condition are highlighted,
Fig. 4 schematically shows the apparatus in Fig. 1 in which the possibilities of movement of the table and of the detector associated with the table in a third operating condition are highlighted,
Fig. 5 schematically shows the apparatus in Fig. 1 in which the possibilities of movement of the table and of the detector associated with the table in a fourth operating condition are highlighted,
Fig. 6 shows, on an enlarged scale, a detail of Fig. 5 that better highlights the structure of the articulated arm of the apparatus,
Fig. 7 schematically shows the apparatus in Fig. 1 in which the possibilities of movement of the table and of the detector associated with the table in a fifth operating condition are highlighted,
Fig. 8 schematically shows the apparatus in Fig. 1 with a detector associated with the teleradiography system and the X-ray emitter in the vicinity, i.e., in a sixth operating condition,
Fig. 9 shows the possibilities of movement of the detector associated with the teleradiography system in Fig. 8,
Fig. 10 schematically shows a patient table suitable for the apparatus in Fig. 1 mounted to an alternative base to that shown in Fig. 1,
Fig. 11 schematically shows a second embodiment of the radiological apparatus according to the present invention, the essential difference with respect to the apparatus in Fig. 1 consisting of the frame which is in part fixed to a sidewall and in part to a floor, and
Fig. 12 schematically shows a third embodiment of the radiological apparatus according to the present invention, the essential difference with respect to the apparatus in Fig. 1 consisting the frame which is in part fixed to a ceiling and in part to a floor.

### Detailed description of the invention

Both this description and these drawings are to be considered only for illustrative purposes and thus not limiting; therefore, the present invention may be implemented according to other and different embodiments; furthermore, it should be borne in mind that these drawings are schematic and simplified.

In Fig. 1 reference numeral 1 indicates as a whole an embodiment of a radiological apparatus according to the present invention.

Basically, apparatus 1 consists of four parts; a first part comprises a frame 9A, an articulated arm 4 and an X-ray emitter 3; a second part comprises a table 2 for the patient, or patient table, a base 7 and a container 52 for an X-ray detector 51; a third part comprises a so-called "teleradiography system" with a column 8 and a container 56 for an X-ray detector 51; a fourth part comprises an electronic control unit 6 of apparatus 1; the same reference numeral 51 is used for both the detector associated with the table 2 and that associated with the column 8, since apparatus 1 could be equipped with only one detector alternatively placed in these two locations or with two identical detectors.

Unit 6 is shown only very schematically; in particular, the connections to the other parts of the apparatus and to the corresponding components are not shown; unit 6 will typically be a computerized unit, i.e. comprising at least an electronic processor in which appropriate programs to control the various electronic, electrical, mechanical and pneumatic components of the apparatus and for generating X-ray images (possibly sequences of radiological images) are loaded. It is worth mentioning here that the detectors of the radiological apparatus are connected to its electronic control unit to transfer the captured images; such a connection may be achieved via a cable; alternatively, such a connection may be achieved wirelessly.

It is clear that such an apparatus can generate radiological images both with the patient in a lying position (in particular lying on table 2) as well as with the patient in a standing position (in particular standing near column 8).

The first X-ray detector 51 is inserted and secured in a container 52, so-called "potter"; container 52 is mounted to a carriage 53 so as to translate horizontally with respect to carriage 53; carriage 53 is mounted to table 2 so as to translate horizontally with respect to table 2; these assemblies are such that the container 52 (and therefore also the detector 51) is always located below the level of the floor of table 2.

Table 2 is mounted to a base 7 so as to translate vertically with respect to base 7; base 7 in Fig. 1 consists of a single element, provided with a foot 71 for supporting it on a floor, located at one end of table 2.

The second X-ray detector 51 is inserted and secured in a container 56, so-called "potter"; container 56 is mounted to a carriage 57 so as to rotate (in particular according to a first vertical rotation axis and according to a second horizontal rotation axis) with respect to carriage 57; carriage 57 is mounted to column 8 so as to translate vertically with respect to column 8.

Column 8 is provided with a foot 81 for supporting it on a floor.

Frame 9A comprises a first frame portion 9A1, in particular in the form of a rectangular box (longer than its width), fixed to a ceiling and a second frame portion 9A2, in particular in the form of a rectangular box (longer than its width), mounted to the first frame portion 9A1; portion 9A2 can translate with respect to portion 9A1 in the direction of the length of portion 9A1; it should be noted that, in Fig. 1, the direction of the length of portion 9A1 is perpendicular to the direction of portion 9A2; it should also be noted that, in Fig. 1, the direction of the length of portion 9A1 corresponds to the direction of the length of table 2.

Arm 4 is mounted, at one end thereof, to the frame portion 9A1; the arm can translate with respect to portion 9A2 in the direction of the length of portion 9A2.

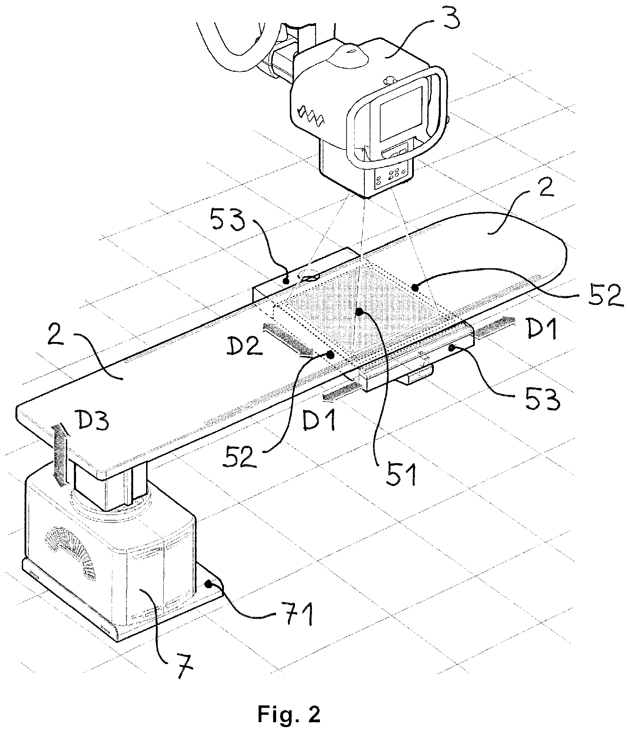

In Fig. 2 the possible movements of the container 52 with respect to table 2 and of the table 2 with respect to base 7 are highlighted; table 2 is horizontal and can translate with respect to base 7 only in a vertical direction D3 so as to adjust the level of table 2 with respect to the floor; carriage 53 can translate with respect to table 2 only in a direction D1 parallel to the length of table 2; container 52 can translate with respect to carriage 53 only in a direction D2 parallel to the width of table 2; therefore, container 52 can only translate according to two horizontal directions perpendicular to each other with respect to table 2.

The translation of carriage 53 extends from approximately 25% of table 2 to approximately 90% of table 2, and thus extends for approximately 2/3 of the length of table 2.

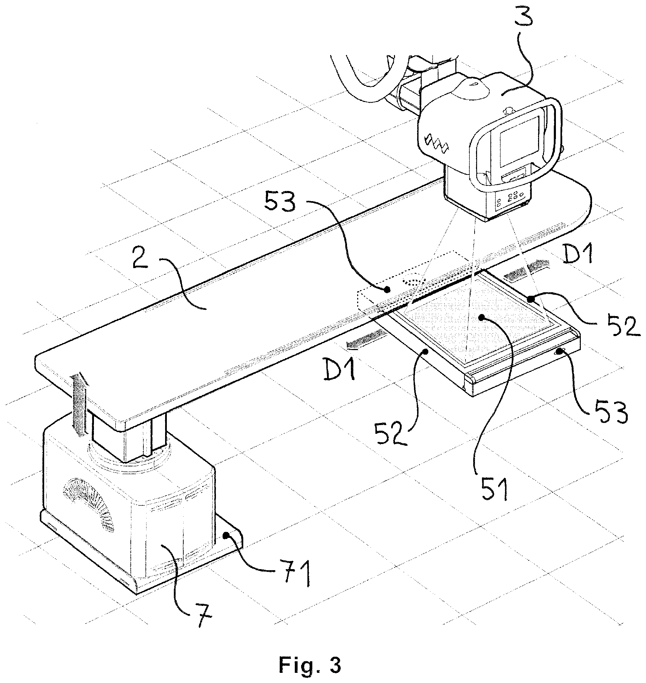

The translation of container 52 is such that detector 51 moves from a position in which it is located exactly at (and below) table 2 (see Fig. 2) to a position completely lateral to (and below) table 2 (see Fig. 3).

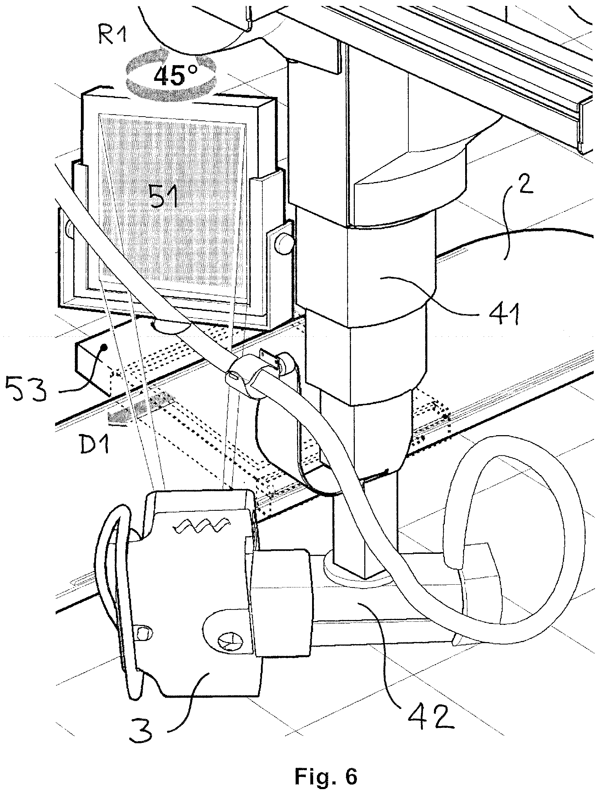

Arm 4 of the embodiment in Fig. 1 comprises a first rectilinear and extendible (in particular telescopic) arm portion 41 and a second rectilinear arm portion 42, as can be seen in Fig. 6; in this embodiment, portion 42 is not extendible, but in alternative embodiments it could be extendible (in particular telescopic). Portion 42 is mounted to one end of portion 41 perpendicular to portion 41; portion 42 is adapted to rotate with respect to portion 41 only about the axis of portion 41. Emitter 3 is mounted to one end of portion 42 so as to rotate only about the axis of portion 42.

The X-ray emitter 3 can be moved manually by the operator by means of a handle with which it is provided, in order to bring it to the position required by the desired radiological image; such a manual movement may cause the translation of portion 9A2 and/or the translation of the whole arm 4 and/or the extension/contraction of portion 41 and/or the rotation of portion 42 and/or the rotation of emitter 3. Alternatively, such a displacement may be carried out by means of motor drivers present in apparatus 1 and a push button keypad included in unit 6. It is also provided that part of such a displacement is carried out automatically by apparatus 1 under the control of unit 6; in fact, according to commands provided by the operator to unit 6 (through a convenient man-machine interface), the (future) position of emitter 3 required by the radiological image may be determined by unit 6 in an approximate manner, the present position of the emitter is known to unit 6, and therefore the required approximate displacement can be calculated by unit 6 and achieved by means of the motor drivers present in apparatus 1; the "fine" positioning of emitter 3 is then achieved by the operator (manual or motorized movement) according to the exact positioning of the patient.

The most important and innovative aspect of the apparatus is the automatic movement of the detector.

In the embodiment in Fig. 1, detector 51 is automatically moved under the control of unit 6. To this end, the radiological apparatus 1 comprises:
- sensors adapted to detect the actual movement of emitter 3, and
- actuators adapted to automatically move (directly or indirectly) detector 51;
   such that the detector 51 is aligned with emitter 3.

The term "alignment" refers, in particular, not only to the fact that the X-ray beam emitted by emitter 3 hits detector 51, but also, advantageously, to the fact that the beam axis coincides with the center of detector 51 and/or is perpendicular thereto. The presence of the sensors that detect the actual movement of the emitter (and therefore its actual position and its actual orientation) is important because the emitter can be moved in various ways (manual movement, motorized movement, automatic movement), and especially is manually "adjusted".

In the embodiment in Fig. 1, detector 51 associated with table 2 moves automatically according to two horizontal directions D1 and D2 perpendicular to each other; however, according to simpler embodiments, the automatic movement according to only one horizontal direction could be provided, for example the direction D1 parallel to the length of table 2. It is worth noting that, in the operating condition of Fig. 3, i.e. with the detector completely lateral with respect to the patient plane (operating condition which could be specifically requested by the operator), the detector can automatically move only according to direction D1.

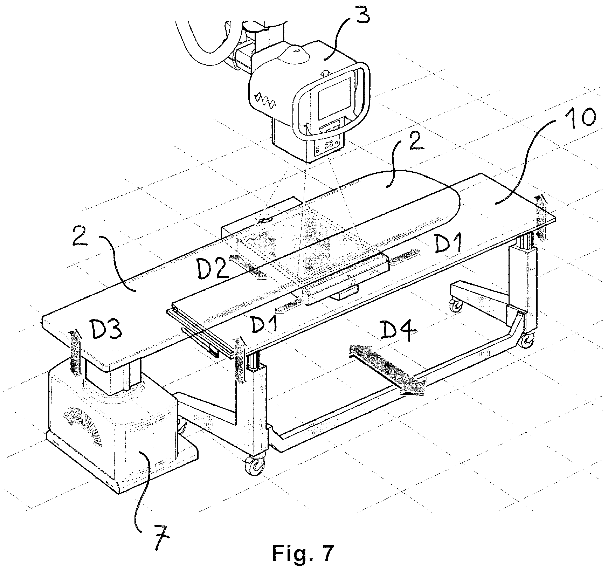

A particular operating condition of the apparatus in Fig. 1 is shown in Fig. 7; the patient is on a movable stretcher 10 which is brought adjacent to table 2 with a patient lying thereon (not shown in the figure), typically with a translational movement in direction D4; the plane of the stretcher can be superimposed in whole, in part or not at all on the patient table and the radiological images can be generated from the apparatus in the same way (i.e. with automatic movement of the X-ray detector) as takes place with the patient lying on the patient table; the table is conveniently lowered thanks to the base so as not to interfere with the stretcher plane.

Unit 6 is connected to the sensors and actuators (a first actuator serves to translate the carriage 53 with respect to table 2 and a second actuator serves to translate the container 52 with respect to carriage 53), and is adapted to obtain the actual movement of emitter 3 from the sensors and consequently to control the actuators such that detector 51 is aligned with emitter 3.

The unit 6 of apparatus 1 is arranged to achieve the movement of the detector 51 such that it follows (possibly with a slight delay) the movement of emitter 3; this takes place both when the movement of the emitter is manual and when it is motorized (possibly also during its possible automatic movement). Alternatively, the logical control unit may be arranged to achieve the movement of the detector such that alignment between detector and emitter is (automatically) achieved according to a user command (for example, an "align detector and emitter" command) and/or when the emitter is stationary.

As already mentioned, apparatus 1 is very flexible and provides various operating conditions in addition to those shown in Fig. 1, Fig. 2, Fig. 3 and Fig. 7.

In Fig. 4, container 52 (together with detector 51) is inserted in a fork 54 and is in a vertical and straight position alongside table 2 (i.e. parallel thereto when viewed from above); fork 54 is provided with a pin that is inserted in a hole (seen in Fig. 2, for example) of carriage 53; due to the sliding of carriage 53 in the direction D1, the automatic alignment of detector 51 can be obtained with respect to emitter 3. In this case, unit 6 can automatically reach the position shown in Fig. 4 and the operator should simply horizontally move the emitter 3, such that it is aligned with the patient's body part for which a radiological image is desired.

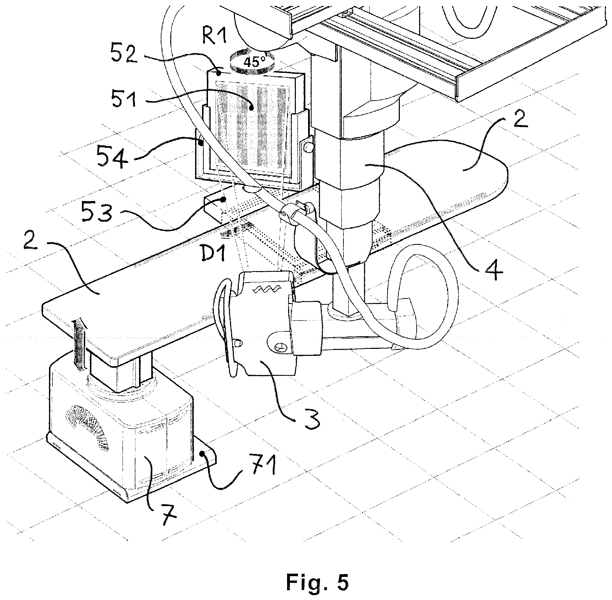

In Fig. 5, container 52 (together with detector 51) is inserted in fork 54, but the fork is mounted to the carriage 53 so as to be inclined, for example, by 45° with respect to the condition of Fig. 4; also in this case, due to the sliding of carriage 53 in direction D1, the automatic alignment of detector 51 can be obtained with respect to the emitter 3. Also in this case, unit 6 can automatically reach the position shown in Fig. 5 such that the operator must simply horizontally move the emitter 3 such that it is aligned with the patient's body part for which a radiological image is desired.

In order for unit 6 to optimally behave when carrying out the automatic alignment, in the embodiment described herein, sensors adapted to detect whether the container 52 is mounted to carriage 53 in a horizontal position (as shown in Fig. 2, Fig. 3 and Fig. 7) or in a vertical position (as in Fig. 4 and Fig. 5) are provided; furthermore, such sensors are able to detect whether the vertical mounting is straight or inclined (possibly the extend of the inclination); therefore, there is a predetermined number of positions (for example: horizontal, vertical straight, vertical inclined at +45°, vertical inclined at 45°); unit 6 is arranges to recognize in which position detector 51 is.

In the embodiment described herein, fork 54 (along with container 52 and detector 51) can rotate neither manually nor automatically with respect to carriage 53. However, according to an alternative solution, such a rotation (see arrow R1 in Fig. 5), or a different rotation, can be automatically achieved under the control of unit 6 to allow automatic alignment of the detector and of the emitter; in this case, for example, the rotation axis is vertical. An automatic rotation of the detector can be combined with an automatic translation.

Another possibility of generating radiological images (possibly sequences of radiological images) by means of the radiological apparatus according to the present invention, in particular by means of the apparatus in Fig. 1, is provided by the "teleradiography system"; this possibility is conceptually independent from that previously described provided by means of the "patient table"; also in this case, a frame, an arm and an X-ray emitter are required.

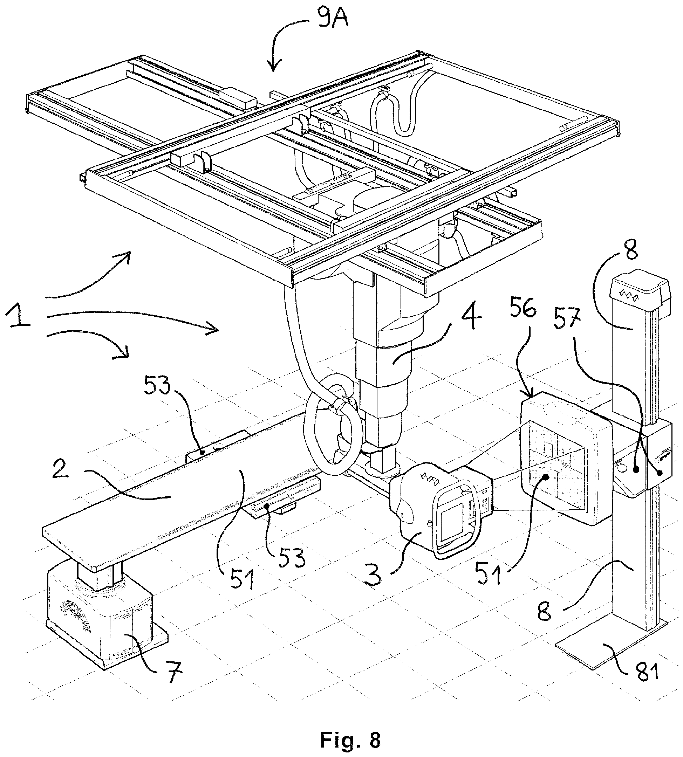

The apparatus in Fig. 1 but arranged to generate radiological images by means of the "teleradiography system" is shown in Fig. 8; in this figure, the X-ray emitter 3 is in the vicinity of column 8 and, more specifically, of container 56 with the detector 51 therein.

In order to achieve this operational condition, frame 9A and arm 4 have been conveniently moved; such movements may have been manual or motorized; it is also included that upon a user command (for example a "generate radiological image with teleradiography system" command) which can be provided by the operator to unit 6 (through a convenient man-machine interface), the unit controls the motor drivers present in the apparatus so as to reach the condition shown in Fig. 8 (or an equivalent one); the "fine" positioning of emitter 3 can then be achieved by the operator (manual or motorized movement) according to the exact positioning of the patient.

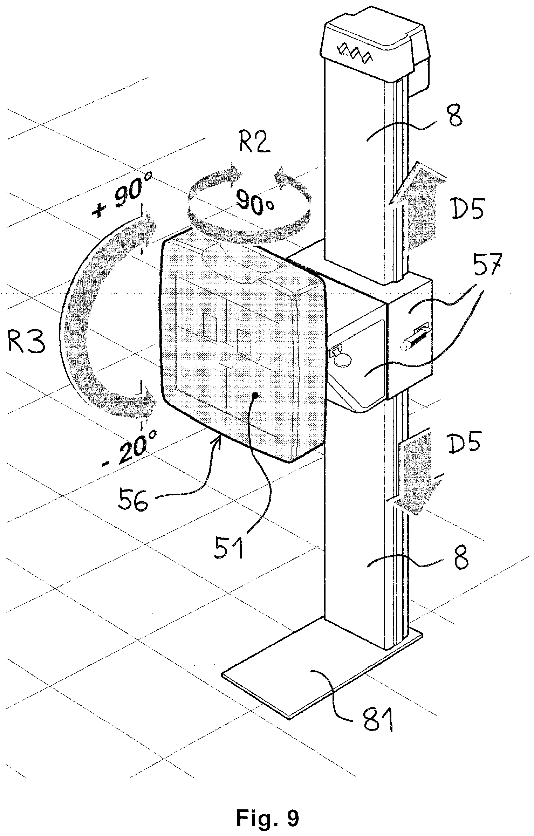

The "teleradiography system" according to the present invention will be described in more detail below with the aid of Fig. 9.

The detector 51 of the "teleradiography system" is inserted and secured in the container 56, so-called "potter"; container 56 is mounted to carriage 57 so as to rotate with respect to carriage 57 (for example, an appropriate joint, in particular a motorized joint, is included); carriage 57 (in particular a motorized carriage) is mounted to column 8 so as to translate with respect to column 8; in particular, the rotation of container 56 is twofold: according to a first vertical rotation axis (see the arrows R2 in the figure) and according to a second horizontal rotation axis (see the arrows R3 in the figure); the translation of carriage 57 is vertical (see the arrows D5 in the figure).

The X-ray detector of the "teleradiography system" also allows automatic alignment with the X-ray emitter, similarly to the X-ray detector of the "patient table"; naturally, this is obtained due to and, in particular, under the control of the electronic control unit of the radiological apparatus.

The automatic movement of detector 51 may include the translation of carriage 57 and/or the first rotation of container 56 and/or the second rotation of container 57.

A particularly simple and effective solution includes that the rotations of the container are carried out manually by the operator and that the only automatic movement is the translation of carriage 57 to allow automatic alignment of said detector and of said emitter; it is also possible that the possible angular positions of container 57 are in a predefined number and achieved manually through operator maneuvers.

In order for unit 6 to optimally behave when carrying out the automatic alignment, sensors adapted to detect the angular position of container 56 and/or the vertical position of carriage 57 may be provided; this is particularly advantageous if such positions are in a predefined number and achieved manually with operator maneuvers; in this case, unit 6 is arranged to know in which position the detector 51 of the "teleradiography system" is located.

In general, it may be advantageous to include a sensor in container 52 and/or in container 56 adapted to detect whether it contains an X-ray detector; in this case, unit 6 is arranged to know the corresponding sensor status.

The embodiment just described may be modified in several ways.

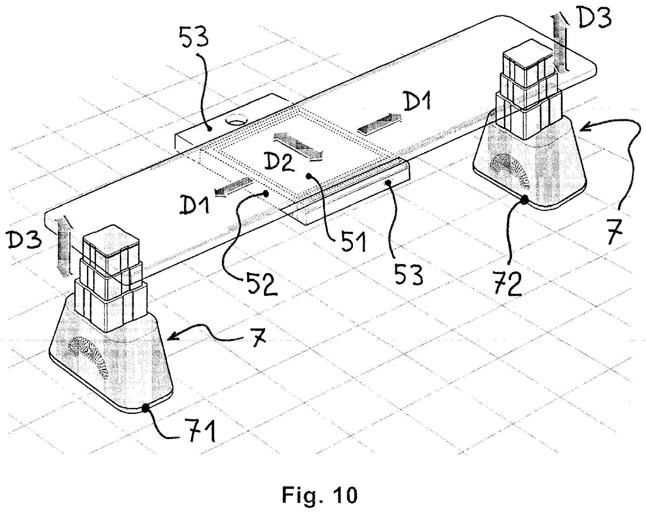

For example, the patient table 2 may be mounted on a base 7 consisting of two elements, provided with two feet 71 and 72, respectively, for supporting it on a floor, as shown in Fig. 10; in this case, a first element (and the first foot 71) is located at a first end of table 2 and a second element (and the second foot 72) is located at a second end of table 2.

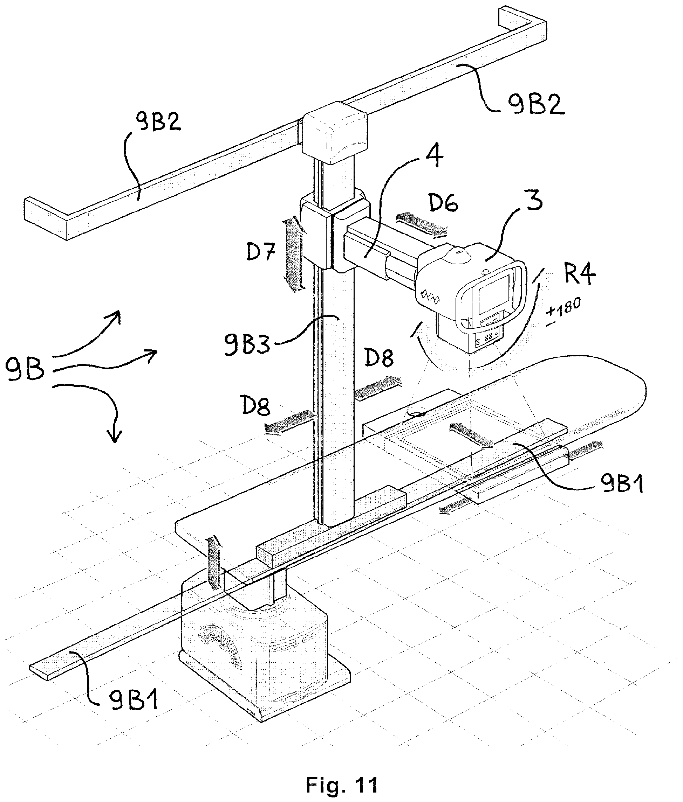

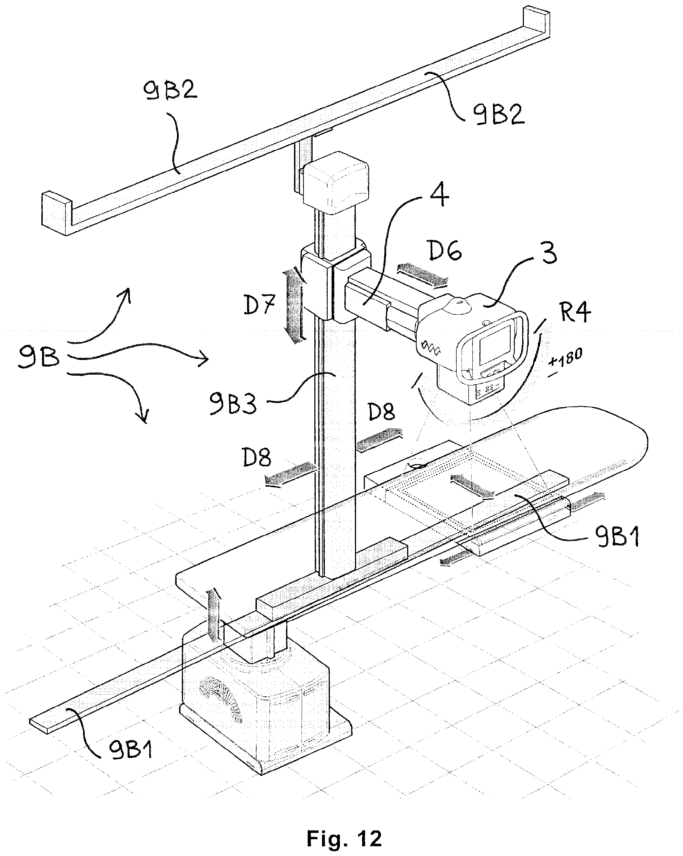

Alternative embodiments to that in Fig. 1 are partially shown in Fig. 11 and Fig. 12; in these two embodiments, patient table and base are identical to those in Fig. 1 and have not been depicted as if they were "transparent"; no "teleradiography system" is included. Obviously, the automatic alignment function of detector 51 with respect to emitter 3 is present.

These two embodiments are very similar to each other and differ in the frame.

Emitter 3 is mounted to a first end of the rectilinear arm 4 in a rotatable manner with respect to the axis of the arm itself (see the arrows R4 in the figure); arm 4 is extendible in a direction D6 corresponding to the axis of arm 4; at the second end of arm 4 is a carriage.

A frame 9B is adapted to support the arm 4 at one end thereof; frame 9B comprises a column 9B3, a first crosspiece 9B1 and a second crosspiece 9B2; the first crosspiece 9B1 is adapted to be horizontally secured at the bottom; the second crosspiece 9B2 is adapted to be horizontally secured at the top; column 9B3 is vertically mounted to the first crosspiece 9B1 and slidably to the second crosspiece 9B2 (two sliding blocks are included, respectively); arm 4 is slidably mounted to the column 9B3 (one sliding block is included).

In the frame in Fig. 11, the second crosspiece 9B2 is adapted to be secured at the top to a wall, while in the frame in Fig. 12, the second crosspiece 9B2 is adapted to be secured to a ceiling.

In both frames, the first crosspiece 9B1 is adapted to be secured to a floor; alternatively, such a crosspiece may be adapted to be secured down a wall.

As can be understood from the foregoing description, the apparatus solution according to the present invention provides:
- possibility to perform all types of radiological examinations,
- possibility to rapidly and easily switch from one radiographic projection to another,
- minimum mechanical constraints in the execution of the various projections,
- reduced manual maneuvering of the various components of the apparatus,
- possibility to use the apparatus in both a "routine" radiology department as well as an "accident and emergency" department,
- high productivity both in the case of "routine" examinations as well as in the case of "accident and emergency" examinations.

As can be understood from the foregoing description, the apparatus solution according to the present invention has undoubted advantages:
- productivity is significantly increased due to the high flexibility of the apparatus which covers all radiological requirements through both manual and automatic management of detector placement,
- it allows the execution of required examinations without moving the patient, even if lying on a stretcher,
- it renders the execution time of examinations much faster, with apparent savings in staff costs and reduction in patient waiting times,
- reducing the time of exposure to X-rays, it provides greater safety conditions for patients and operators,
- it allows the customization of the detectors provided according to the specific requirements of individual purchasers of the apparatus,
- the physical effort associated with the execution of the examination is significantly reduced and increases the productivity of the apparatus.

## Claims

1. A radiological apparatus (1) comprising a patient table (2), an X-ray emitter (3) mounted to an arm (4) and adapted to be moved, an X-ray detector (51) associated with said patient table (2), and an electronic control unit (6) adapted to control at least said emitter (3) and said detector (51), and comprising further:
- sensors adapted to detect the actual movement of said emitter (3),
- at least one actuator adapted to automatically move said detector (51) at least in a horizontal direction (D1, D2);
wherein said emitter (3) is adapted to undergo a displacement by an operator, both manually by means of a handle with which it is provided and in a motorized manner;
wherein said electronic control unit (6) is connected to said sensors and to said at least one actuator, and is adapted to obtain from said sensors the actual movement of said emitter (3) in both cases of motorized and manual displacement of said emitter (3), and therefore to control said at least one actuator so that, during normal use of the apparatus, said detector (51) is automatically aligned with said emitter (3) before starting capturing a radiological image or a plurality of radiological images.

2. An apparatus (1) according to Claim 1, wherein said patient table (2) is mounted to a base (7), said base (7) being adapted to move said patient table (2) only in a vertical direction (D3), so that the position of said patient table (2) can be only vertically adjusted.

3. An apparatus (1) according to Claim 1 or 2, comprising two actuators adapted to automatically move said detector (51), in a first horizontal direction (D1) and in a second horizontal direction (D2), respectively, wherein said second direction (D1) is perpendicular to said first direction (D2), and
wherein said electronic control unit (6) is connected to said sensors and to said first and second actuators, and is adapted to obtain from said sensors the movement of said emitter (3), and therefore to control said first and second actuators so that said detector (51) is aligned with said emitter (3).

4. An apparatus (1) according to any one of the preceding Claims, comprising at least one support (52, 53, 54; 56, 57) adapted to support said detector (51) in a horizontal or vertical position, and adapted to be moved (D1, D2, D5; R1, R2, R3), in particular under the control of said electronic control unit (6), in order to allow the automatic alignment of said detector (51) and said emitter (3).

5. An apparatus (1) according to Claim 4, wherein said support (52, 53, 54; 56, 57) has a predetermined number of fixed positions for said detector (51) and is adapted to notify the position taken by said detector (51) to said electronic control unit (6) in order to allow the automatic alignment of said detector (51) and said emitter (3).

6. An apparatus (1) according to Claim 4 or 5, comprising a first support (52, 53, 54) associated with said patient table (2) and a second support detached and remote from said patient table (2).

7. An apparatus (1) according to any one of the preceding Claims, wherein said logic control unit (6) is arranged so that, during normal use of the apparatus, it determines movements of said detector (51) such that said detector (51) follows said emitter (3) before starting capturing a radiological image or a plurality of radiological images.

8. An apparatus (1) according to any one of the preceding Claims, wherein said arm (4) is adapted to be moved in a manual and/or motorized manner.

9. An apparatus (1) according to any one of the preceding Claims, wherein said arm (4) is adapted to be automatically moved under the control of said electronic control unit (6) in order to allow the coarse automatic alignment of said detector (51) and said emitter (3).

10. An apparatus (1) according to any one of the preceding Claims, wherein said arm (4) comprises a first extendible arm portion (41), a second arm portion (42) perpendicularly mounted to one end of said first portion (41) with respect to said first portion (41), wherein said second portion (42) is adapted to rotate about said first portion (41), wherein said emitter (3) is rotatably mounted to one end of said second portion (3).

11. An apparatus (1) according to Claim 1, wherein said automatic alignment follows said operator displacement.

## Patentansprüche

1. Röntgengerät (1), aufweisend
- eine Patientenliege (2),
- einen Röntgenstrahler (3), der an einem Arm (4) befestigt und derart ausgestaltet ist, um bewegt zu werden,
- einen Röntgendetektor (51), der mit der Patientenliege (2) verbunden ist, und
- eine elektronische Steuerungseinheit (6), die geeignet ist, zumindest den Strahler (3) und den Detektor (51) zu steuern,
weiter aufweisend:
- Sensoren, die geeignet sind, die tatsächliche Bewegung des Strahlers (3) zu erfassen;
- zumindest einen Aktuator, der ausgestaltet ist, um den Detektor (51) zumindest in horizontaler Richtung (D1, D2) automatisch zu bewegen;
wobei der Strahler (3) ausgestaltet ist, durch den Anwender, sowohl manuell mittels eines Griffs, mit dem er ausgestattet ist, als auch motorisiert bewegt zu werden; wobei die elektronische Steuerungseinheit (6) mit den Sensoren und dem zumindest einen Aktuator verbunden und geeignet ist, in beiden Fällen, dem motorisierten und dem manuellen Verschieben des Strahlers (3), von den Sensoren die tatsächliche Bewegung des Strahlers (3) zu erhalten, um damit den zumindest einen Aktuator so zu steuern, dass, bei normalem Gebrauch des Geräts, der Detektor (51) automatisch mit dem Strahler (3) ausgerichtet ist, bevor begonnen wird, ein Röntgenbild oder eine Vielzahl von Röntgenbildern aufzunehmen.

2. Gerät (1) gemäß Anspruch 1, wobei die Patientenliege (2) auf einer Basis (7) montiert ist und die Basis (7) ausgestaltet ist, die Patientenliege (2) nur in einer vertikalen Richtung (D3) zu bewegen, so dass die Patientenliege (2) nur in vertikaler Richtung verstellt werden kann.

3. Gerät (1) gemäß Anspruch 1 oder 2, aufweisend zwei Aktuatoren, die geeignet sind, den Detektor (51) automatisch in einer ersten horizontalen Richtung (D1) bzw. einer zweiten horizontalen Richtung (D2) zu bewegen, wobei die zweite horizontale Richtung (D2) senkrecht zur ersten horizontalen Richtung (D1) ist und wobei die elektronische Steuerungseinheit (6) mit den Sensoren und dem ersten und dem zweiten Aktuator verbunden und geeignet ist, von den Sensoren die Bewegung des Strahlers (3) zu erhalten und somit den ersten und den zweiten Aktuator so zu steuern, dass der Detektor (51) mit dem Strahler (3) ausgerichtet ist.

4. Gerät (1) gemäß einem der vorigen Ansprüche, aufweisend zumindest einen Träger (52, 53, 54; 56, 57), der den Detektor (51) in einer horizontalen oder vertikalen Position trägt und geeignet ist, insbesondere unter Kontrolle der elektronischen Steuerungseinheit (6), bewegt zu werden (D1, D2, D5; R1, R2, R3), um die automatische Ausrichtung des Detektors (51) mit dem Strahler (3) zu ermöglichen.

5. Gerät (1) gemäß Anspruch 4, wobei der Träger (52, 53, 54; 56, 57) eine vorbestimmte Anzahl fixer Positionen für den Detektor (51) aufweist und geeignet ist, die vom Detektor (51) eingenommene Position an die elektronische Steuerungseinheit (6) zu übermitteln, um die automatische Ausrichtung des Detektors (51) mit dem Strahler (3) zu ermöglichen.

6. Gerät (1) gemäß Anspruch 4 oder 5, aufweisend einen ersten Träger (52, 53, 54), der mit der Patientenliege (2) verbunden ist und einen von der Patientenliege (2) getrennten und entfernten zweiten Träger.

7. Gerät (1) gemäß einem der vorigen Ansprüche, wobei die logische Steuerungseinheit (6) so eingerichtet ist, dass sie bei normalem Gebrauch des Geräts Bewegungen des Detektors (51) ermittelt, so dass der Detektor (51) dem Strahler (3) folgt, bevor begonnen wird, ein Röntgenbild oder eine Vielzahl von Röntgenbildern aufzunehmen.

8. Gerät (1) gemäß einem der vorigen Ansprüche, wobei der Arm (4) geeignet ist, manuell und / oder motorisiert bewegt zu werden.

9. Gerät (1) gemäß einem der vorigen Ansprüche, wobei der Arm (4) geeignet ist, automatisch unter der Kontrolle der elektronischen Steuerungseinheit (6) bewegt zu werden, um eine grobe automatische Ausrichtung des Detektors (51) mit dem Strahler (3) zu ermöglichen.

10. Gerät (1) gemäß einem der vorigen Ansprüche, wobei der Arm (4) einen ersten ausfahrbaren Armabschnitt (41) und einen zweiten ausfahrbaren Armabschnitt (42) aufweist, der senkrecht bezüglich des ersten Abschnitts (41) an einem Ende des ersten Abschnitts (41) befestigt ist, wobei der zweite Abschnitt (42) in der Lage ist, um den ersten Abschnitt (41) zu rotieren, und der Strahler (3) drehbar an einem Ende des zweiten Teils (42) befestigt ist.

11. Gerät (1) gemäß einem der vorigen Ansprüche, wobei die automatische Ausrichtung der Bewegung durch den Anwenders folgt.

## Revendications

1. Appareil radiologique (1) comprenant une table pour patient (2), un émetteur de rayons X (3) monté sur un bras (4) et conçu pour être déplacé, un détecteur de rayons X (51) associé à ladite table pour patient (2), et une unité de commande électronique (6) conçue pour commander au moins ledit émetteur (3) et ledit détecteur (51), et comprenant en outre :
- des capteurs conçus pour détecter le déplacement réel dudit émetteur (3),
- au moins un actionneur conçu pour déplacer automatiquement ledit détecteur (51) au moins dans une direction horizontale (D1, D2) ;
dans lequel ledit émetteur (3) est conçu pour être soumis à un déplacement par un opérateur, à la fois manuellement au moyen d'une poignée dont il est doté et de manière motorisée ;
dans lequel ladite unité de commande électronique (6) est connectée auxdits capteurs et audit au moins un actionneur, et est conçue pour obtenir desdits capteurs le déplacement réel dudit émetteur (3) dans les deux cas de déplacement motorisé et manuel dudit émetteur (3), et par conséquent pour commander ledit au moins un actionneur de sorte que, en utilisation normale de l'appareil, ledit détecteur (51) soit automatiquement aligné avec ledit émetteur (3) avant le début de la capture d'une image radiologique ou d'une pluralité d'images radiologiques.

2. Appareil (1) selon la revendication 1, dans lequel ladite table pour patient (2) est montée sur une base (7), ladite base (7) étant conçue pour déplacer ladite table pour patient (2) uniquement dans une direction verticale (D3), de sorte que la position de ladite table pour patient (2) puisse être réglée uniquement verticalement.

3. Appareil (1) selon la revendication 1 ou 2, comprenant deux actionneurs conçus pour déplacer automatiquement ledit détecteur (51) respectivement dans une première direction horizontale (D1) et dans une seconde direction horizontale (D2), dans lequel ladite seconde direction (D1) est perpendiculaire à ladite première direction (D2), et
dans lequel ladite unité de commande électronique (6) est connectée auxdits capteurs et auxdits premier et second actionneurs, et est conçue pour obtenir desdits capteurs le déplacement dudit émetteur (3), et par conséquent pour commander lesdits premier et second actionneurs de sorte que ledit détecteur (51) soit aligné avec ledit émetteur (3).

4. Appareil (1) selon l'une quelconque des revendications précédentes, comprenant au moins un support (52, 53, 54; 56, 57) conçu pour supporter ledit détecteur (51) dans une position horizontale ou verticale, et conçu pour être déplacé (D1, D2, D5; R1, R2, R3), en particulier sous la commande de ladite unité de commande électronique (6), afin de permettre l'alignement automatique dudit détecteur (51) et dudit émetteur (3).

5. Appareil (1) selon la revendication 4, dans lequel ledit support (52, 53, 54; 56, 57) possède un nombre prédéterminé de positions fixes pour ledit détecteur (51) et est conçu pour notifier la position prise par ledit détecteur (51) à ladite unité de commande électronique (6) afin de permettre l'alignement automatique dudit détecteur (51) et dudit émetteur (3).

6. Appareil (1) selon la revendication 4 ou 5, comprenant un premier support (52, 53, 54) associé à ladite table pour patient (2) et un second support détaché et distant de ladite table pour patient (2).

7. Appareil (1) selon l'une quelconque des revendications précédentes, dans lequel ladite unité de commande électronique {6) est agencée de sorte que, en utilisation normale de l'appareil, elle détermine les déplacements dudit détecteur (51) de sorte que ledit détecteur (51) suive ledit émetteur (3) avant le début de la capture d'une image radiologique ou d'une pluralité d'images radiologiques.

8. Appareil (1) selon l'une quelconque des revendications précédentes, dans lequel ledit bras (4) est conçu pour être déplacé de manière manuelle et/ou motorisée.

9. Appareil (1) selon l'une quelconque des revendications précédentes, dans lequel ledit bras (4) est conçu pour être déplacé automatiquement sous la commande de l'unité de commande électronique (6) afin de permettre l'alignement automatique approximatif dudit détecteur (51) et dudit émetteur (3).

10. Appareil (1) selon l'une quelconque des revendications précédentes, dans lequel ledit bras (4) comprend une première partie de bras extensible (41), une seconde partie de bras (42) montée perpendiculairement sur une extrémité de ladite première partie (41) par rapport à ladite première partie (41), dans lequel ladite seconde partie (42) est conçue pour tourner autour de ladite première partie (41), dans lequel ledit émetteur (3) est monté en rotation sur une extrémité de ladite seconde partie (42).

11. Appareil (1) selon la revendication 1, dans lequel ledit alignement automatique suit ledit déplacement de l'opérateur.
